# EUROPEAN PATENT APPLICATION

(11) **EP 4 670 723 A1**
(43) Date of publication of application: **31.12.2025**
(21) Application number: 24306032.4
(22) Date of filing: 26.06.2024
(51) Int. Cl.: A61K 31/7076, A61K 31/52, A61K 31/519, A61K 45/06, A61P 19/02

(54) **PICLIDENOSON FOR USE IN THE TREATMENT OF OSTEOARTHRITIS IN NON-HUMAN MAMMALS**

(71) Applicant: Vetbiolix, 59120 Loos (FR); Can-Fite Biopharma Ltd, 49170 Petach Tikva (IL)
(72) Inventor: The designation of the inventor has not yet been filed
(74) Representative: Cabinet Beau de Loménie

(57) **Abstract**

The present invention relates to piclidenoson for use in a method of treating osteoarthritis in a non-human mammal, wherein said piclidenoson is administered orally in a daily dose from 300 to 2000 µg/kg body weight.

## Description

### FIELD OF THE INVENTION

The present invention relates to a novel veterinary use of piclidenoson, a known adenosine A3 receptor (A3AR) agonist, in the treatment of osteoarthritis.

### BACKGROUND

Osteoarthritis, known in the past as degenerative arthritis, is the most common form of arthritis in animals. It is a joint disease that occurs after abnormality or damage of joints or without joint damage. The disease involves the deterioration of cartilage in the joints. Over time, the cartilage, covering the ends of bones in a joint, begins to break down and may wear away entirely, and the bones will rub together, causing pain. Due to pain in a joint, the surrounding muscle is used less, and muscle strength is thus weakened.

The usual symptoms of osteoarthritis are stiffness, limitation of motion, pain and joint deformity and affected joints display edema, hot flashes and abnormal enlargement of joints.

Osteoarthritis itself does not greatly affect one's life, but chronic osteoarthritis sustaining for a long period of time causes pain and deformity of the joints and thus reduces the quality of life. In particular, osteoarthritis in the knees is known as a major cause of chronic disability.

Various drugs and treatment methods have been developed and used for the treatment of osteoarthritis in human. The main goals of the treatment are to relieve pain, maintain the functions of the joints and prevent disability due to the functional disorder of the joints.

Currently there is no known medical treatment to reverse the effects of this cartilage damage in animals. Rather the therapies for osteoarthritis are directed mainly towards treating the symptoms. In this regard, osteoarthritis has been treated using anti-inflammatory substances of the corticosteroid type (such as hydrocortisone and Betamethasone), which function to inhibit prostaglandin synthesis, as well as with a large number of nonsteroidal anti-inflammatory drugs (NSAIDs, such as diclofenac, aspirin and ibuprofen), which have an analgesic as well anti-inflammatory effect. However, due to their serious side effects, these drugs are used with special caution.

US patent n°10265337, filed by Can-Fite, discloses a method for treating osteoarthritis in a subject, comprising administering a therapeutically effective amount of piclidenoson, wherein the exemplified therapeutically effective amount is between 1 to 100 µg/kg body weight.

Reference [1] discloses oral administration of piclidenoson (CF101) to monoidoacetate (MIA) experimental rat models in a dose of 100 µg/kg body weight twice a day (i.e. bid). The authors showed that CF101 deregulated the NF-kappaB signaling pathway involved in the pathogenesis of osteoarthritis. CF101 induced apoptosis of inflammatory cells and acted as a cartilage protective agent, which suggested that it would be a suitable candidate drug for the treatment of OA.

Based on the results observed in MIA experimental rat models model, Can-Fite conducted a clinical trial phase 2 study to evaluate efficacy and safety of 1000 µg (14.5 µg/kg) twice daily of piclidenoson administered orally for 12 weeks to patients with osteoarthritis of the knee. However, the clinical trial was discontinued due to a lack of efficacy.

### SUMMARY OF THE INVENTION

The Applicant entered a clinical trial to evaluate efficacy and safety of 100 µg/kg and 500 µg/kg twice a day of piclidenoson administered orally for 90 days to dogs with osteoarthritis (data not disclosed). Despite the data available in MIA experimental rat models, the Applicants have surprisingly shown that a dose of 100 µg/kg twice a day do not provide evidence for efficacy of piclidenoson in the treatment of osteoarthritis in dogs.

However, the Applicants have surprisingly found that notwithstanding the clinical liabilities of the piclidenoson in humans, and notwithstanding the clinical liabilities of the piclidenoson in dogs, piclidenoson is efficacious in the treatment of osteoarthritis in non-human mammals in a dose much higher than 100 µg/kg twice a day. Contrary to what was observed at a dose of 100 µg/kg twice a day, the Applicants have surprisingly shown that piclidenoson is particularly efficient in a daily dose from 300 to 2000 µg/kg body weight.

Therefore, the present invention relates to piclidenoson for use in a method of treating osteoarthritis in a non-human mammal, wherein said piclidenoson is administered orally in a daily dose from 300 to 2000 µg/kg body weight.

### DETAILLED DESCRIPTION OF THE INVENTION

### Definitions

The term "piclidenoson", also known as "IB-MECA", "CF101" or *"*1-Deoxy-1-[6-[[(3-iodophenyl)methyl]amino]-9H-purin-9-yl]-N-methyl-β-D-ribofuranuronamide*"*, is an adenosine A3 receptor (A3AR) agonist having the CAS N°152918-18-8.

The term "treat" or "treatment" comprises treating osteoarthritis to reverse diseases symptoms, preventing the development of osteoarthritis, as well as managing and/or ameliorating osteoarthritis or one or more symptoms thereof. Thus, treatment refers to administering a therapeutically effective amount of piclidenoson to achieve a desired therapeutic effect. The desired therapeutic effect may include, without being limited thereto, improving motility of the subject, decrease in swelling and tenderness of the joints, slowing or preventing the deterioration of the joints and the surrounding tissue, slowing any irreversible damage caused by a chronic stage of osteoarthritis, increasing the time period of the remission between acute attacks of the disease, lessening of the severity of or curing osteoarthritis, or providing more rapid recovery form osteoarthritis, as well as decreasing any one of the following symptoms: stiffness, pain and joint deformity, joint edema, hot flashes and abnormal enlargement of joints or preventing the manifestation of such symptoms before they occur. In the context of the present invention treatment also includes prevention of the development of osteoarthritis (e.g. in subjects having high disposition of developing the disease) as well as reversal of damage caused to cartilage as a result of the disease.

The term "pharmaceutical composition" is intended to mean a combination of the active agent(s), together or separately, with a pharmaceutically acceptable carrier as well as other additives. The carrier may at times have the effect of the improving the delivery or penetration of the active ingredient to the target tissue, for improving the stability of the drug, for slowing clearance rates, for imparting slow-release properties, for reducing undesired side effects etc. The carrier may also be a substance that stabilizes the formulation (e.g. a preservative), for providing the formulation with an edible flavor, etc. For examples of carriers, stabilizers and adjuvants, see E. W. Martin, REMINGTON'S PHARMACEUTICAL SCIENCES, MacK Pub Co (June, 1990).

The term "pharmaceutically acceptable carrier" in the context of the present invention denotes any one of inert, non-toxic materials, which do not react with piclidenoson and which can be added to formulations as diluents, carriers or to give form or consistency to the formulation. The pharmaceutically acceptable excipients must be adapted to the non-human mammal to be treated. For example, dogs and cats cannot tolerate any product with mint flavouring. Another example, xylitol has been shown to be toxic to dogs producing liver damage and anaemia.

### Invention

The present invention relates to piclidenoson for use in a method of treating osteoarthritis in a non-human mammal, wherein said piclidenoson is administered orally in a daily dose from 300 to 2000 µg/kg body weight.

In some embodiments, piclidenoson is administered in a daily dose from 400 to 1600 µg/kg body weight, such as from 500 to 1500 µg/kg body weight, such as from 800 to 1200 µg/kg body weight, for example in a daily dose of 1000 µg/kg body weight.

In some embodiments, piclidenoson is administered between once and a few times a day, preferably once or twice a day. For example, piclidenoson may be administered in a dose from 150 to 1000 µg/kg body weight twice a day, such as from 200 to 800 µg/kg body weight twice a day, such as such as from 250 to 750 µg/kg body weight twice a day, such as such as from 400 to 600 µg/kg body weight twice a day, for example 500 µg/kg body weight twice a day.

In some embodiments, piclidenoson is in a form suitable for oral administration, such as in a form of a pharmaceutical composition suitable for oral administration. Typical examples of carriers suitable for oral administration include (a) liquid solutions, where an effective amount of piclidenoson is dissolved in diluents, such as water, saline, natural juices, alcohols, syrups, etc.; (b) capsules (e.g. the ordinary hard- or soft-shelled gelatin type containing, for example, surfactants, lubricants, and inert fillers), tablets, lozenges (wherein piclidenoson is in a flavor, such as sucrose or piclidenoson is in an inert base, such as gelatin and glycerin), and troches, each containing a predetermined amount of piclidenoson as solids or granules; (c) powders; (d) suspensions in an appropriate liquid; (e) suitable emulsions; (f) liposome formulation; and others.

Compositions for oral administration in the present invention may conveniently be presented in unit dosage form (e.g. tablets and sustained release capsules) and may be prepared by any method known in the art of pharmacy. Compositions may be presented as discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion and as a bolus etc.

Alternatively, the composition may be nutraceutically formulated, together with food, supplements, chew sticks or other veterinary products including toothpastes and dental prophylaxis pastes. A large number of taste masking and flavour enhancing additives (palatants) are commercially available for feline and canine products, from companies such as AFB International, Firmenich and SPF.

In some embodiments, the non-human mammal is treated for at least 2 weeks, such as at least 3 weeks, such as at least 1 month, such as at least 2 months, such as at least 3 months, such as at least 4 months, such as at least 5 months, such as at least 6 months.

In some embodiments, the non-human mammal has osteoarthritis and is treated with another drug selected from methotrexate (MTX), steroids and NSAIDS (non-steroidal anti-inflammatory drugs).

With regard to compositions for oral administration (e.g. tablets and capsules), the term excipient includes: binding agents, for example syrup, acacia, gelatine, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatine, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Palatants, as mentioned above for nutraceuticals can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the piclidenoson in a free-flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally coated or scored and may be formulated so as to provide slow or controlled release of the active agent.

Other formulations suitable for oral administration include lozenges comprising piclidenoson, usually a veterinarily palatable carbohydrate and acacia or tragacanth and pastilles comprising piclidenoson in an inert base such as gelatine and glycerin, or sucrose and acacia.

In some embodiments, piclidenoson is administered in a unit dosage form, preferably as capsule or tablets.

As noted above, the therapeutic use of piclidenoson may at times be in combination with other drugs such as MTX, steroids, NSAIDS (e.g. ibuprofen, diclofenac, narpoxen), COX2 inhibitors (e.g. celcoxib), serotonin and norepinephrine reuptake inhibitors (e.g. duloxetine), opioids (e.g. tramadol), IL1 receptor antagonists (e.g. anakinra), TNF inhibitors (e.g. etanercept, certolizumab), JAK inhibitors (e.g. rituximab). In such a combination treatment the other drug and piclidenoson may be given to patients at the same time or at different times, depending on the dosing schedule of each of the drugs. MTX, for example, is typically given to patients once weekly at doses ranging between 5 and 25 mg, each weekly dose, either orally or parenterally. Piclidenoson is typically given at a more frequent dosing schedule, for example once or twice daily.

In some embodiments, the non-human mammal is selected from the group consisting of a feline and a canine, preferably selected from the group consisting of a cat and a dog.

### BRIEF DESCRIPTION OF THE FIGURES

Figure 1: LOAD before and after treatment in 100 µg/kg bid group. Values are expressed as mean ± SD. Statistical Significance 90-days vs Inclusion was assessed using non-parametric Wilcoxon matched pairs rank test. p value is noted on the graph.
Figure 2: LOAD before and after treatment in 500 µg/kg bid group. Values are expressed as mean. Statistical Significance 90-days vs Inclusion assessed using non-parametric Wilcoxon matched pairs rank test. p value is noted on the graph.
Figure 3: LOAD evolution in each individual patient treated with 100 µg/kg bid.
Figure 4: LOAD evolution in each individual patient treated with 500 µg/kg bid.
Figure 5: Mean LOAD evolution in 100 µg/kg bid group. Values are expressed as mean ± SD (n=6). ns = not significant, * p<0.05 according to Friedman test for multiple comparisons.
Figure 6: Mean LOAD Evolution in 500 µg/kg bid group. Values are espressed as mean ± SD (n=9). ns: not significant, * p<0.05; ** p<0.01 according to Friedman test for multiple comparisons.
Figure 7: Proportion of patients with or without clinically meaningful change in LOAD at Day-90 vs Inclusion. According to [2], a minimal LOAD change of > 4 points is considered as a clinically meaningful disease evolution.
Figure 8: Comparison of piclidenoson at 500 µg/kg bid effects on LOAD in dog patients with osteoarthritis vs effects of Ligament Surgery ([2]) on LOAD in dog patient with ligament rupture.

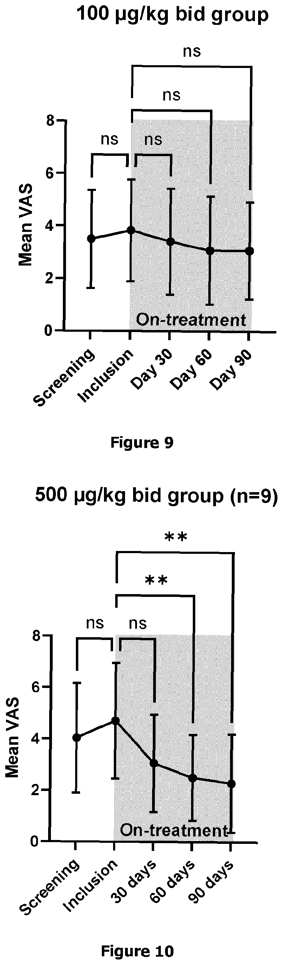

Figure 9: Mean VAS evolution in 100 µg/kg bid group. Values are expressed as mean ± SD (n=6). ns = not significant, * p<0.05 according to Friedman test for multiple comparisons.
Figure 10: Mean LOAD Evolution in 500 µg/kg bid group. Values are expressed as mean ± SD (n=9). ns: not significant, * p<0.05; ** p<0.01 according to Friedman test for multiple comparisons.
Figure 11: Proportion of patients with or without clinically meaningful change in VAS in the two group of treatment.
Figure 12: NRS1 (Lameness) stages distribution at inclusion and after 90 days in 100 µg/kg bid group. ns: not significant according to Fisher exact test.
Figure 13: NRS2 (Pain) stages distribution at inclusion and after 90 days in 100 µg/kg bid group. ns = not significant according to Fisher exact test.
Figure 14: NRS1 (Lameness) stages distribution at inclusion and after 90 days in 500 µg/kg bid group. Ns = not significant according to exact Fisher exact test.
Figure 15: NRS2 (Pain) stages distribution at inclusion and after 90 days in 500 µg/kg bid group. * p<0.05 according to Fischer exact test.
Figure 16: Study design to evaluate effect of 90 days treatment with CF-101 at 100 µg/kg bid and 500 µg/bid in dog patients with osteoarthritis (OA).

### EXAMPLES

### Example 1: Effects of 90 days treatment with CF-101 at 100 µg/kg bid and 500 µg/bid in dog patients with osteoarthritis (OA)

### Study design

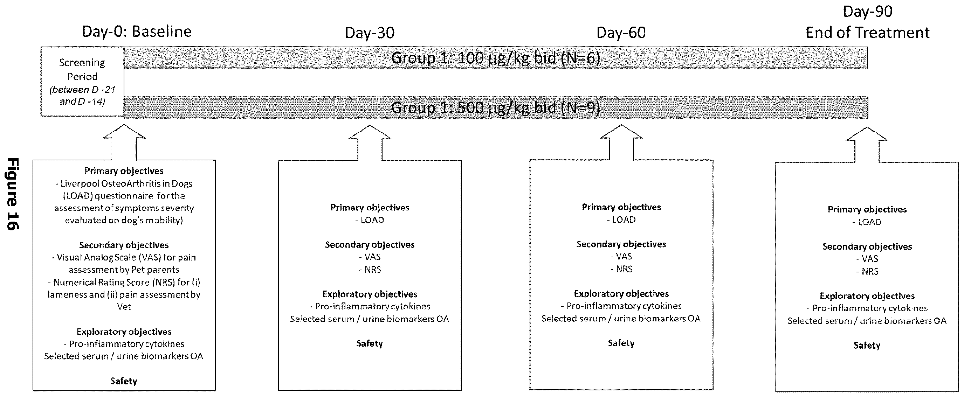

The study design can be schematically resumed in Figure 16.

This was a multicentric, non-comparative, randomized proof of concept study in which 15 dogs with osteoarthritis (OA) were treated with piclidenosone (VBX1000) for 90 days.

At inclusion (Day-0) dog were randomly assigned to two treatment groups:
- Group 1: dogs were treated with 100 µg/kg piclidenososne (VBX1000) BID (n=9)
- Group 2: dogs were treated with 500 µg/kg of VBX1000 BID (n=6) Piclidenosonoe (VBX1000) was administrated twice-daily (morning and evening) with a meal for 90 days. Two intermediate visits were included for clinical examination and for calculating OA scores.

The primary objective of this study was to assess the efficacy of piclidenosone to reduce OA symptoms severity after 90 days of treatment. For that purpose, OA symptoms and motility were scored by the owner using Liverpool Osteoarthritis in Dogs (LOAD) questionnaire at Day-0, Day-30, Day-60 and Day-90.

Secondary Objectives included: i) assessment of piclidenosone efficacy to reduce OA related pain severity assessed by the owner using Visual Analog Scale (VAS); ii) assessment by the veterinarian of piclidenosone efficacy to reduce pain severity using Numerical Rating Score 1 (NRS1); iii) assessment by the veterinarian of piclidenosone efficacy to reduce OA associated lameness associated OA using Numerical Rating Score (NRS2); iv) evaluation of tolerance of VBX013 in client-owned dogs with OA.

### Dog Patient population

- Inclusion criteria
   ∘ The owner approved and signed the informed consent form prior to the participation of his/her dog in the study;
   ∘ Dog older than 18 months;
   ∘ Dog with a Body Condition score lower than 8 (on a 9 point-scale);
   ∘ Dog with a symptomatic unifocal or multifocal osteoarthritis, characterized by the presence of osteophyte on at least one site on radiographic views;
   ∘ LOAD mobility total score of ≥ 11 at visit V2 (Day 0).
- Exclusion criteria
   ∘ Animals intended for breeding or known to be pregnant or lactating;
   ∘ Dog with severe OA which requires concomitant treatment;
   ∘ Presence of complete cranial cruciate ligament rupture;
   ∘ Breeds at risk of MDR1 gene mutation (MDR1 genetic test required before screening of a dog from known affected breed: Collie, Border Collie, Australian and English Shepherd, German Shepherd, Long-Haired Whippet, Shetland Sheepdog);
   ∘ History of orthopaedic surgery including complete cranial cruciate ligament rupture surgically treated with Tibial Tuberosity Advancement (TTA) or Tibial Plateau Leveling Osteotomy (TPLO) or elbow debridement within 3 months prior to inclusion;
   ∘ Presence of musculoskeletal lesions (e.g., glycogenosis, primary muscular dystrophy) or untreated neoplasia prior to inclusion;
   ∘ Presence of concurrent systemic disease such as neurologic, hepatic, renal or cardiac disease;
   ∘ Dog under physical therapy (including acupuncture) for orthopaedic indication unless a 1-week wash-out period is applied prior to visit V1 (between D-21 and D-14);
   ∘ Dog treated with methotrexate;
   ∘ Dog treated with nonsteroidal anti-inflammatory drugs unless a 2-weeks wash-out period is applied prior to visit V1 (between D-21 and D-14);
   ∘ Dog that received mavacoxib or enflicoxib within 3 months prior to visit V1 (between D-21 and D-14);
   ∘ Dog treated with short-acting corticosteroids (i.e., hydrocortisone) unless a 2-weeks wash-out period is applied prior to visit V1 (between D-21 and D-14);
   ∘ Dog treated with intermediate-acting corticosteroids (i.e., prednisone, prednisolone, methylprednisolone) unless a 4-weeks wash-out period is applied prior to visit V1 (between D-21 and D-14);
   ∘ Dog treated with long-acting corticosteroids (i.e., dexamethasone) unless a 12-weeks wash-out period is applied prior to visit V1 (between D-21 and D-14);
   ∘ Dog treated with any intra-articular injection in the joint under study within 2 months prior to visit V1 (between D-21 and D-14);
   ∘ Dog treated with a subcutaneous injection of bedinvetmab unless a 4-week wash-out periods is applied prior to visit V1 (between D-21 and D-14);
   ∘ Dog treated with OA modifying diet and/or food supplements (i.e., glycosaminoglycans, glucosamine and chondroitin sulphate or other) unless its administration has been regular during the month preceding visit V1 (between D-21 and D-14) and will be continued during the study duration;
   ∘ Dog unable to walk on a leash.

### Concomitant treatments

- Authorized treatments:
   ∘ OA modifying diet and/or food supplements (i.e. glycosaminoglycans, glucosamine and chondroitin sulphate) will be continued during the study duration in dogs that have received them regularly during minimum 1 month preceding the visit V1;
   ∘ Any concomitant treatments deemed necessary to provide adequate supportive care except for those listed as a forbidden treatment;
   ∘ Sedation for radiographic views included in the protocol with the Investigator's choice of sedation protocol;
- Non authorized treatments:
   ∘ Oral and injectable NSAIDs;
   ∘ Oral and injectable corticosteroids;
   ∘ Methotrexate;
   ∘ Bedinvetmab;
   ∘ Intra-articular administration of any treatment;
   ∘ Physiotherapy;
   ∘ Any analgesic treatment including alternative forms of pain relief (acupressure, acupuncture, chiropractic manipulation) except sedation procedure for radiographic views included in the protocol;
   ∘ OA modifying diet and/or food supplements (i.e. glycosaminoglycans, glucosamine and chondroitin sulphate) for dogs that have not received them regularly during minimum 1 month preceding the visit V1.

### Results

Throughout the study, piclidenoson was well tolerated with no treatment related SAE and no death.

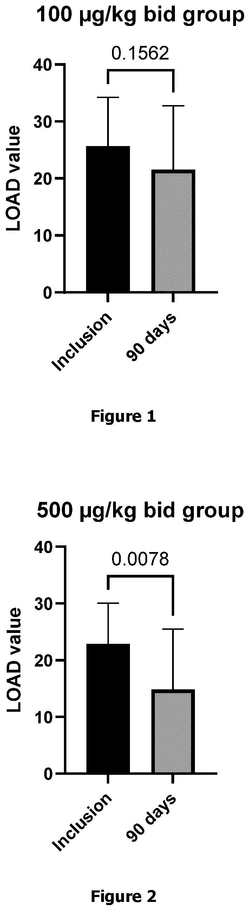

As shown in Figure 1, after 90 days of treatment with 100 µg/kg bid the primary endpoint was not met. In this group, LOAD score at the end of the treatment period was not significantly reduced as compared to baseline value (21.5t11.3 at Day-90 vs 25.7±8.6 at Day-0, N=6; ns).

In contrast, in the group treated with 500 µg/kg bid for 90 days the primary of the study was met (Figure 2). In this group, there was a statistically significant decrease in LOAD score after 90 days of treatment (14.9±10.6 at Day-90 vs 22.9±7.2 at Day-0, N=9; p<0.01).

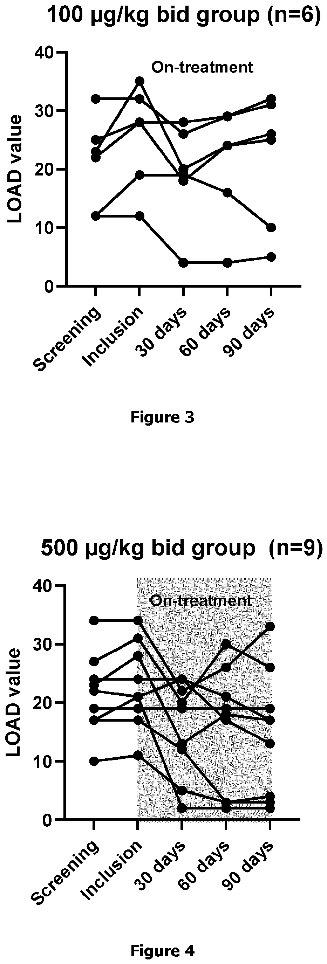

Figure 3 and Figure 4, shows temporal evolution of LOAD score in each individual patients included in group 1 treated at 100 µg/kg bid (Figure 3) and in group 2 treated at 500 µg/kg bid during the 90-day treatment period (Figure 4).

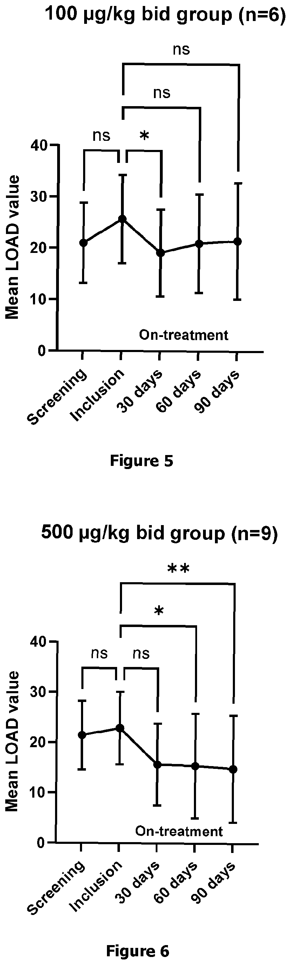

Figure 5 and figure 6 illustrates temporal evolution of mean LOAD score in group 1 at 100 µg/kg bid and in group 2 at 500 µg/kg bid. A transient decrease in LOAD score was in group 1 was observed at Day-30 which totally disappeared at Day-60 and Day-90 (Figure 5). In contrast in group 2 treated with 500 µg/kg bid a time-dependent decrease in LOAD score was observed reaching statistical significance from Day-60 to the end of the treatment period (Figure 6).

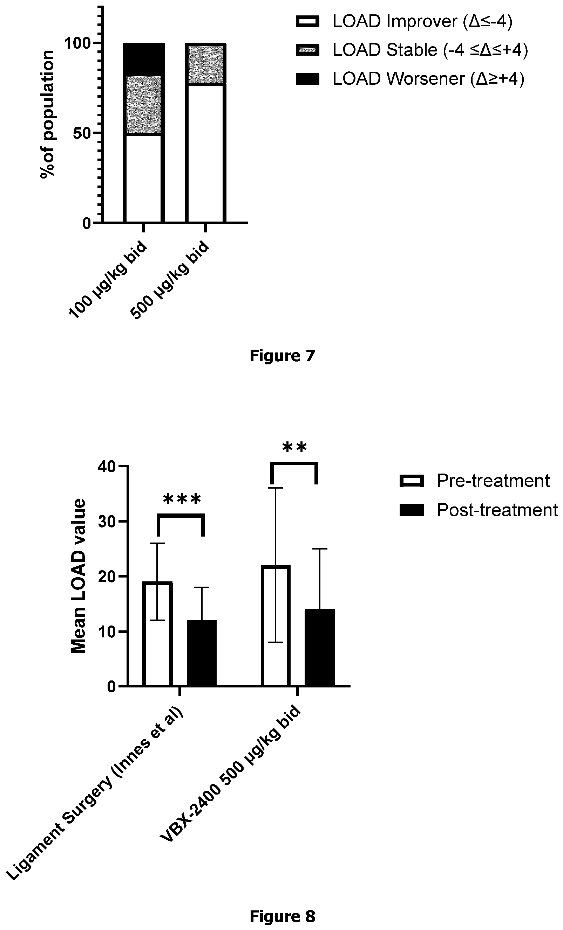

As proposed by Iness et al. [2], a decrease in LOAD score ≥4 is considered as a clinically meaningful amelioration of motility in osteoarthritic dogs. As shown in Figure 7 the proportion of patient experiencing clinically meaningful improvement of LOAD score was higher in the 500 µg/kg bid group as compared to the 100 µg/kg bid.

Further arguing for clinically meaningful efficacy of piclidenosone on motility the change in LOAD score obtained after 90 days of treatment with 500 µg/kg bid was comparable to that obtained after ligament surgery in dogs with ligament rupture (Figure 8).

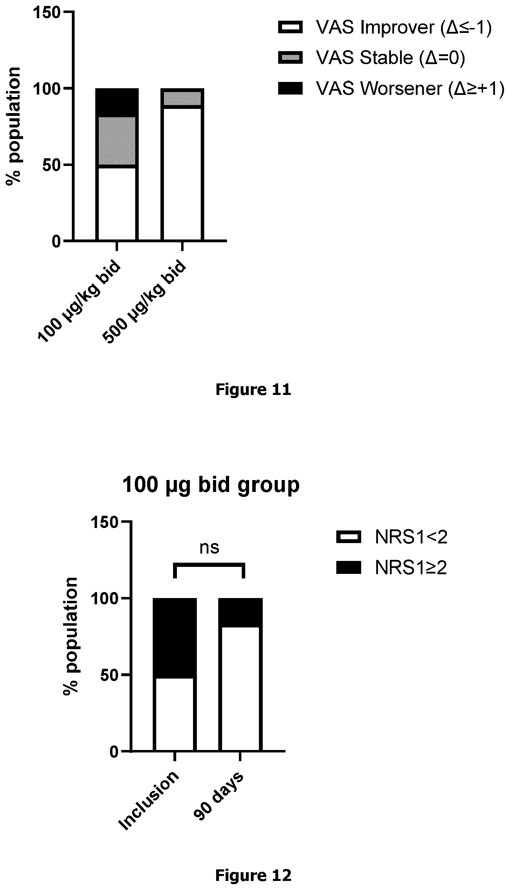

Similar to what was observed on the LOAD motility score, there was a dose- and time-dependent improvement of the pain score (Visual Analog Score or VAS) throughout the treatment period. Whereas VAS was not significantly improved in the 100 µg/kg bid group, a significant decrease was obtained in the 500 µg/kg bid. At this dose, the beneficial effects of piclidenosone treatment on VAS was rapid after treatment initiation, reaching statistical significance from Day-60 up to the end of the treatment period. Aligned with a dose dependent effect of piclidenosone on VAS, the proportion of patients showing VAS improvement (Δ≤-1) at the end of the treatment period was superior in the 500 µg/kg bid group as compared to the 100 µg/kg bid group (Figure 11).

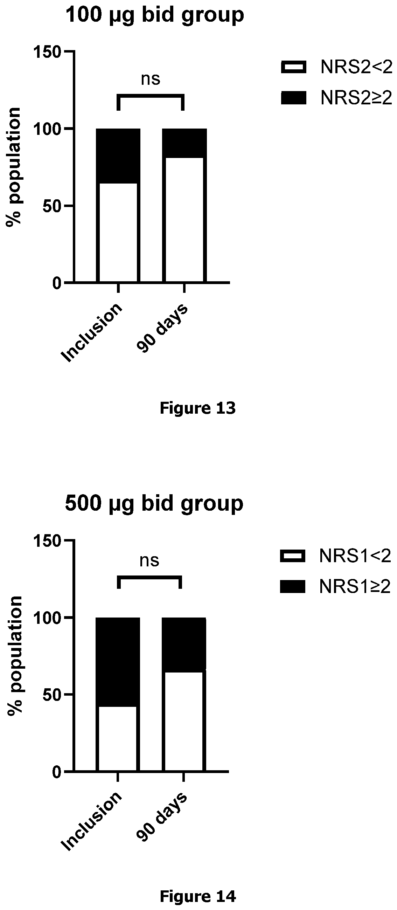

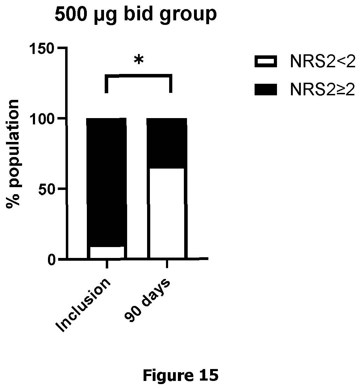

Figure 12-15 show the effect vs baseline of piclidenosone treatment on the two components of the NRS score (NRS1 lameness score and NRS2 pain score). The 90-day treatment with a low dose of 100 µg/kg bid provoked non-significant increase in the proportion of patient with low NRS1 (Figure 12) and NRS2 (Figure 13). In the 500 µg/kg bid group, the effect of piclidinosone treatment on the proportion of patients with low scores reached statistical significance for NRS2 (Figure 15) but not for NRS1 (Figure 14).

### Conclusion

Together these results demonstrate that repeated oral administration of piclidenosone at dose ≥ 500 µg/kg bid can be used to improves clinical symptoms of osteoarthritis in dog.

### Example 2: Use of allometric factors to compare Active Dose (AD) in dog vs human

| **Species** | **Bid Dose** | | **Activity** | **Factor D/R to H** | **Human ED** | **Factor H to D** | **Dog ED** |
|---|---|---|---|---|---|---|---|
| | µg | µg/kg | Yes/No | | µg/kg | | µg/kg |
| **Human** | 2000 | 29 | Yes | | 29 | 1,80 | 51 |
| | 4000 | 57 | Yes | | 57 | 1,80 | 103 |
| Rat | | 100 | Yes | 0,162 | 16 | 1,80 | 29 |
| **Dog** | | 100 | No | 0,541 | 54 | 1,80 | 100 |
| | | 500 | Yes | 0,541 | 271 | 1,80 | 500 |

| | | | | | | | |
|---|---|---|---|---|---|---|---|
| D to H = dog to human, R to H= rat to human; H to D = human to dog. | | | | | | | |

### Results

From results of clinical Phase 2 trials in human patients, the Active Dose in Human might be set at 2000 mg or 29 µg/kg (assuming body weight of 70 kg). The Dog Equivalent dose (DED) can be calculated at 51 µg/kg.

In our proof-of-concept trial in dog with osteoarthritis the Active Dose was 500 µg/kg corresponding to HED = 271 µg/kg.

### Conclusion

The Active Dose of 500 µg/kg in dog is about 10 folds higher than the Active Dose in human (271 µg/kg vs 29 µg/kg when expressed as HED or 500 µg/kg vs 51 µg/kg when expressed as DED).

### Example 3: Use of allometric factors to extrapolate Active Dose in different animal species

| **Human ED** | **Factor Hto D** | **Dog ED** | **Factor H to C** | **Cat ED** |
|---|---|---|---|---|
| µg/kg | | µg/kg | | µg/kg |
| 29 | 1,8 | 51 | 2,3 | 66 |
| 57 | 1,8 | 103 | 2,3 | 131 |
| 16 | 1,8 | 29 | 2,3 | 37 |
| 54 | 1,8 | 100 | 2,3 | 124 |
| 271 | 1,8 | 500 | 2,3 | 622 |

The Active Dose of 500 µg/kg in dog is about 10 folds higher than the Active Dose in human (271 µg/kg in dog vs 29 µg/kg in human).

With an Active Dose of 500 µg/kg in dog, the cat equivalent dose is calculated at 622 µg/kg.

### REFERENCES

[1] Bar-Yehuda et al., Induction of an antiinflammatory effect and prevention of cartilage damage in rat knee osteoarthritis by CF101 treatment, 2009, Arthritis Rheum
[2] Innes et al., Minimal clinically-important differences for the 'Liverpool Osteoarthritis in Dogs' (LOAD) and the 'Canine Orthopedic Index' (COI) client-reported outcomes measures, PLoS One, 2023 Feb 2;18(2):e0280912

## Claims

1. Piclidenoson for use in a method of treating osteoarthritis in a non-human mammal, wherein said piclidenoson is administered orally in a daily dose from 300 to 2000 µg/kg body weight.

2. Piclidenoson for use according to claim 1, wherein piclidenoson is administered in a daily dose from 500 to 1500 µg/kg body weight.

3. Piclidenoson for use according to any of the preceding claims, wherein piclidenoson is administered in a daily dose of 1000 µg/kg body weight.

4. Piclidenoson for use according to any of the preceding claims, wherein piclidenoson is administered once or twice a day.

5. Piclidenoson for use according to any of the preceding claims, wherein piclidenoson is administered in a dose from 150 to 1000 µg/kg body weight twice a day.

6. Piclidenoson for use according to any of the preceding claims, wherein piclidenoson is administered in a dose from 250 to 750 µg/kg body weight twice a day.

7. Piclidenoson for use according to any of the preceding claims, wherein piclidenoson is administered in a dose of 500 µg/kg body weight twice a day.

8. Piclidenoson for use according to any of the preceding claims, wherein the non-human mammal is treated for at least two weeks.

9. Piclidenoson for use according to any of the preceding claims, wherein the non-human mammal is treated for at least one month.

10. Piclidenoson for use according to any of the preceding claims, wherein the non-human mammal is treated for at least two months.

11. Piclidenoson for use according to any of the preceding claims, wherein the non-human mammal has osteoarthritis and is treated with another drug selected from methotrexate, steroids and NSAIDS (non-steroidal anti-inflammatory drugs).

12. Piclidenoson for use according to any of the preceding claims, wherein said non-human mammal is selected from the group consisting of a feline and a canine.

13. Piclidenoson for for use according to any of the preceding claims, wherein said non-human mammal is selected from the group consisting of a cat and a dog.

14. Piclidenoson for use according to any of the preceding claims, wherein the piclidenoson is in a form suitable for oral administration, such as in a form of a pharmaceutical composition suitable for oral administration.
